(19) **Europäisches Patentamt / European Patent Office / Office européen des brevets**

(11) **EP 3 766 537 B1**

(12) # EUROPEAN PATENT SPECIFICATION

(45) Date of publication and mention
of the grant of the patent:
**11.06.2025 Bulletin 2025/24**

(21) Application number: **19768013.5**

(22) Date of filing: **12.03.2019**

(51) International Patent Classification (IPC):
**A61M 37/00** *(2006.01)*    **A61F 13/02** *(2024.01)*

(52) Cooperative Patent Classification (CPC):
**A61M 37/0015; A61F 13/0256;** A61M 2037/0023;
A61M 2037/0046; A61M 2037/0061

(86) International application number:
**PCT/JP2019/009866**

(87) International publication number:
**WO 2019/176900 (19.09.2019 Gazette 2019/38)**

(54) **ADHESION MEMBER AND MICRONEEDLE PATCH**

HAFTELEMENT UND MIKRONADELPFLASTER

ÉLÉMENT D'ADHÉRENCE ET TIMBRE À MICRO-AIGUILLES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.03.2018 JP 2018044944**

(43) Date of publication of application:
**20.01.2021 Bulletin 2021/03**

(73) Proprietor: **MEDRx Co., Ltd.
Higashikagawa-shi
Kagawa 769-2712 (JP)**

(72) Inventors:
• **KOBAYASHI, Katsunori
Higashikagawa-shi, Kagawa 769-2712 (JP)**

• **HAMAMOTO, Hidetoshi
Higashikagawa-shi, Kagawa 769-2712 (JP)**

(74) Representative: **Kraus & Lederer PartGmbB
Thomas-Wimmer-Ring 15
80539 München (DE)**

(56) References cited:
EP-A1- 3 251 721          WO-A1-2015/111673
WO-A1-2017/014129        WO-A1-2017/110815
JP-A- 2003 313 330       JP-A- 2003 534 881
JP-A- 2008 522 731       JP-A- 2009 025 065
JP-A- 2009 045 789       JP-A- 2013 155 244
JP-A- 2015 089 442       JP-A- 2015 158 022
JP-A- 2016 210 023

**Description**

TECHNICAL FIELD

The present disclosure relates to an adhesive member. Also, the present disclosure relates to a patch or microneedle patch in which a microneedle array is carried on such an adhesive member, an auxiliary tool for puncturing microneedles into the skin, and the like, which is made of such an adhesive member. In particular, it relates to a microneedle patch which is excellent in fixability to the skin.

BACKGROUND OF THE INVENTION

Transdermal administration of drugs using a microneedle array is an area that has begun to be actively studied in recent years. Then, a practical method of manufacturing a microneedle array has been studied from many companies so far (Patent Documents 1 and 2). Methods of applying a microneedle array to the skin have also been investigated (Patent Documents 3 and 4), and as a mechanism for puncturing microneedles into the skin, methods of injecting or pressing microneedles with the force of a spring, methods of pressing microneedles with the force of a hand, and the like have been investigated.

However, the microneedle array punctured and attached to the skin by the method of injection or pressing with the force of a spring or the method of pressing with the force of a hand has a problem in that the microneedle array floats from the skin due to the repulsive force of the skin, and a sufficient effect of injecting drugs cannot be obtained. In particular, in order to improve the skin puncturability of microneedle array, when a tension is applied to the skin and pulled and the microneedle array is applied, the skin shrinks more remarkably to float up the microneedle array.

PRIOR ART

PATENT DOCUMENT

Patent Document 1 : JP 2009-273872 A
Patent Document 2: JP WO2012-128363 A
Patent Document 3: JP 2010-516337 A
Patent Document 4: JP 2014-042788 A

EP3251721A1 relates to transdermal administration devices used for drug administration.

SUMMARY OF THE INVENTION

PROBLEM TO BE SOLVED BY THE PRESENT INVENTION

The invention is defined in claim 1. Any methods disclosed hereinafter do not form part of the scope of the invention, and are mentioned for illustrative purposes only.

As described above, conventionally, there has been a problem that the microneedle array floats due to contraction of the skin or elasticity of the skin when the microneedle array is ejected or pressed by the force of a spring or pressed by the force of a hand and punctured or applied without applying tension to the skin. Therefore, it has been desired to develop a new patch which can stably maintain the fixation of the microneedle array to the skin.

It is a main problem of the present invention to provide a new microneedle patch which is excellent in fixability to skin or puncturability of microneedles. Another problem of the present invention is to provide an adhesive member therefor.

MEANS FOR SOLVING THE PROBLEMS

In order to solve the above problems, the present inventors have studied an adhesive member in microneedle patches, or a support thereof.

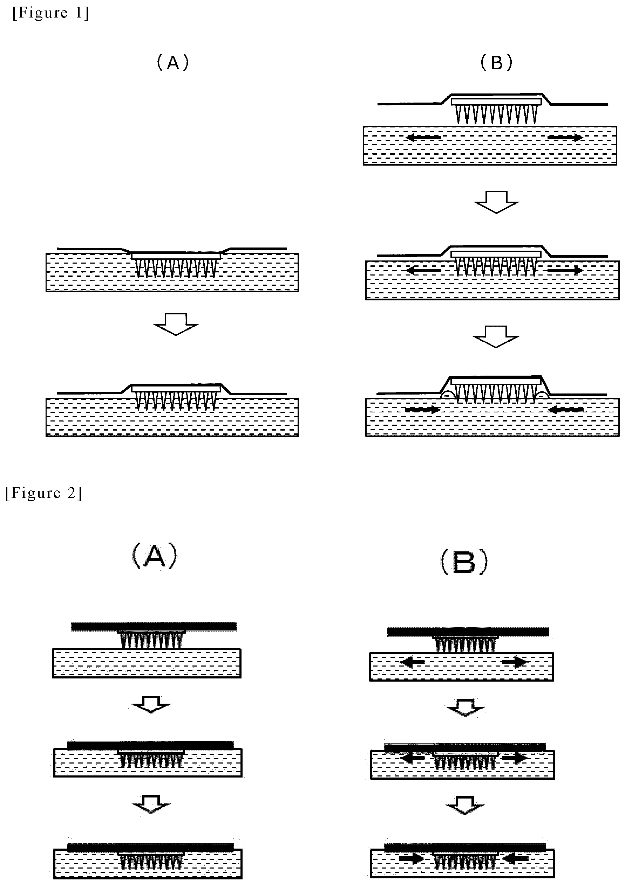

First, if a microneedle array is applied without stretching the skin, the elasticity of the skin causes the microneedle array to float, as shown in Figure 1(A). On the other hand, if a microneedle array is applied after the skin is stretched to enhance the puncture property of microneedles, the microneedle array will float due to the relaxation of the skin after puncturing as shown in Figure 1(B).

The present inventors have found that, if the skin is adhesively fixed with an adhesive member having an appropriate rigidity, floating of the pasted microneedle array can be prevented (see Figure 2).

Based on the above findings, the present inventors have come to complete the present invention.

EFFECT OF THE INVENTION

A patch according to the present invention is excellent for puncturing microneedles or an array thereof into the skin and staying them at the skin.

The state of the skin to be punctured by the microneedle varies greatly depending on the age. For example, as a person becomes a child, adult, and elderly, the stretchability and elasticity of the skin becomes poorer. Therefore, the puncturability of microneedle is also greatly affected. According to the present invention, it is possible to avoid variations in puncture property based on elasticity of the skin while achieving stable puncture property of microneedle and quantitative property of drug administration. Alternatively, according to the present invention, it is possible to avoid a floating phenomenon of microneedle array based on skin elasticity or skin contraction from the skin. As a result, administration of the required amount of drug carried in the microneedle array can be achieved. That is, it is possible to avoid the floating of microneedle or microneedle array from the skin due to repulsion in the direction of puncture of the skin, or to extend the skin to improve puncture, and to avoid the floating of microneedle or microneedle array from the skin after application, so that quantitative drug administration by the microneedle is possible.

BRIEF DESCRIPTION OF THE DRAWINGS

[Fig. 1] Figure 1 represents the condition when a conventional microneedle patch is applied to the skin. The figure (A) represents that when the microneedle patch is applied as it is without stretching the skin, the microneedle array floats due to the elasticity of the skin. The figure (B) represents that when the skin is stretched and the microneedle patch is applied as it is, the skin shrinks and the microneedle array floats. In each figure, the top shows the microneedle patch and the bottom shows the skin, respectively. In the figure (B), the upper figure shows that the skin is tensioned by pulling the skin in the direction of the right and left arrows. The middle figure shows that the microneedles have been punctured and inserted while the skin is pulled. The figure below shows that the skin is not pulled, the skin is shrinking, and the patch is about to shrink.

[Fig. 2] Figure 2 represents the condition when a microneedle patch according to the present invention is applied to the skin. The figure (A) represents that even if the microneedle patch is applied as it is without stretching the skin, the microneedle array does not float due to the elasticity of the skin. The figure (B) represents that, even if the skin is stretched and the microneedle patch is applied as it is, the skin shrinks and the microneedle array does not float. In each figure, the top shows the microneedle patch and the bottom shows the skin, respectively. In the figure (B), the upper figure shows that the skin is tensioned by pulling the skin in the direction of the right and left arrows. The middle figure shows that the microneedles have been punctured and inserted while the skin is pulled. The figure below shows that the skin is not pulled, the skin is shrinking, and the patch is about to shrink.

[Fig. 3] Figure 3 shows an exemplary patch according to the present invention. The figure (a) shows a plan view, and the figure (b) shows a side view in the longitudinal direction.

[Fig. 4] Figure 4 shows an exemplary patch according to the present invention. The figure (a) shows a plan view, and the figure (b) shows a side view in the longitudinal direction.

[Fig. 5] Figure 5 shows an exemplary patch according to the present invention. The figure (d) is a perspective view seen from the concave side (concave view), and the others are perspective views seen from the side hollow portion is raised (convex view).

[Fig. 6] Figure 6 shows an exemplary patch according to the present invention. The figure (a) shows a portion comprising a patch on which a microneedle array is carried. The figure (b) shows a state in which such a portion is carried in the patch. The figure (c) is a perspective view of the patch from the outside.

[Fig. 7] Figure 7 shows an exemplary auxiliary tool according to the present disclosure.

[Fig. 8] Figure 8 shows the use of an auxiliary tool according to the present disclosure.

[Fig. 9] Figure 9 is a top perspective view of an exemplary device according to the present invention. The figure (a) shows one that a member for skin stretching is two, and the figure (b) shows one that a member for skin stretching is four.

[Fig. 10] Figure 10 shows an exemplary device according to the present invention. The figure (a) shows a front view, the figure (b) shows a plan view, and the figure (c) shows a front view when the member for skin stretching is expanded, respectively.

[Fig. 11] Figure 11 shows an exemplary device according to the present invention. The figure (a) shows a front view, the figure (b) shows a rear view (view from the microneedle array side), and the figure (c) shows a cross-sectional view at the top and bottom lines of the figure (b), respectively.

[Fig. 12] Figure 12 shows an exemplary device according to the present invention. The figure (a) left and the figure (b) left show a front view, and the figure (a) right and the figure (b) right show a plan view, respectively.

[Fig. 13] Figure 13 shows a schematic use diagram of a device according to the figure (a) or (b) of Figure 11.

[Fig. 14] Figure 14 represents a schematic diagram of a three-point bending test apparatus for determining rigidity.

[Fig. 15] Figure 15 shows an example of the load curve of the result obtained by the three-point bending test. The vertical axis indicates the load (N) and the horizontal axis indicates the deflection (mm), respectively.

[Fig. 16] Figure 16 represents the apparatus used in Example 2.

[Fig. 17] Figure 17 represents the experimental results in Example 2. The vertical axis represents the stress (N) at the time of pushing, and the horizontal axis represents the stress (MPa•mm$^4$), respectively.

EMBODIMENT FOR CARRYING OUT THE PRESENT INVENTION

Hereinafter is described about the present invention in detail.

1 Adhesive member according to the present disclosure

An adhesive member according to the present disclosure (hereinafter referred to as the "adhesive member of the present disclosure") is characterized in setting up a pressure-sensitive adhesive on one surface of a support and in having a rigidity of 1.3 MPa•mm$^4$ or more.

The "support" in the adhesive member of the present disclosure is not particularly limited, as long as it can be used for medical use and a rigidity of the adhesive member when a pressure-sensitive adhesive is set up is 1.3 MPa•mm$^4$ or more. The support, for example, can include one made of fiber, resin, or metal. Examples of the support made of fiber can include nonwoven fabric and woven fabric, and specific examples of the material can include cotton, rayon, silk, pulp, and chemical fibers such as polyester. Specific examples of the material of the support made of resin can include polyethylene, polypropylene, polyvinyl chloride, acrylic, polyethylene terephthalate, polystyrene, acrylonitrile-butadiene-styrene copolymer, polycarbonate, polyamide, fluororesin, polybutylene terephthalate, and urethane. Specific examples of the material of the support made of metal can include aluminum, stainless steel, and titanium.

The adhesive member of the present disclosure has a rigidity of 1.3 MPa•mm$^4$ or more, preferably in the range of 1.3 to 3400 MPa•mm$^4$, more preferably in the range of 1.3 to 1200 MPa•mm$^4$, and still more preferably in the range of 1.3 to 730 MPa•mm$^4$. When a microneedle array is carried on an adhesive member having a rigidity lower than 1.3 MPa•mm$^4$ to form a microneedle patch, applying such a patch to the skin may lead the microneedle array to float from the skin. When a microneedle array is carried on an adhesive member higher than 3400 MPa•mm$^4$ to form a microneedle patch, the microneedle array does not float from the skin, but the microneedles are hardly stressed, and there is a possibility that the microneedles are not successfully punctured into the skin. Of particular importance here is that the adhesive member has a rigidity of 1.3 MPa•mm$^4$ or more

The rigidity of adhesive member of the present disclosure is appropriately adjusted according to the materials of the support and the pressure-sensitive adhesive, the manufacturing conditions thereof, the thickness thereof, the size thereof, the shape thereof, and the like.

It is preferable that a stress of 15 N or more is applied to a microneedle array, and more preferable that a stress of 20 N or more is applied to a microneedle array.

The "pressure-sensitive adhesive" in the adhesive member of the present disclosure can be used as long as it is for medical use, and known pressure-sensitive adhesives can be used. Specifically, for example, an acrylic adhesive made of an acrylic polymer; a styrene block copolymer such as a styrene-isoprene-styrene block copolymer, and a styrene-butadiene-styrene block copolymer; a rubber-based adhesive such as polyisoprene, polyisobutylene, and polybutadiene; a silicon-based adhesive such as silicon rubber, dimethylsiloxane base, and diphenylsiloxane base; a vinylether-based adhesive such as polyvinylmethylether, polyvinylethylether, and polyvinylisobutylether; a vinylester-based adhesive such as vinyl-acetate-ethylene copolymer; a polyester-based adhesive such as dimethylterephthalate, dimethylisophthalate and dimethylphthalate, made of a carboxylic acid component and a polyhydric alcohol component such as ethylene glycol. Any one of these pressure-sensitive adhesives may be used, or 2 or more of them may be used in combination.

The pressure-sensitive adhesive may be set up on the entire surface of the support, and may be set up on a part thereof. Here, a layer of the pressure-sensitive adhesive in a range in which the pressure-sensitive adhesive is set up on the support is referred to as the "adhesive layer". The pressure-sensitive adhesive may be set up on the support, dividing into 2 or more, and in this case, a plurality of adhesive layers will be present.

The planar shape of the adhesive member of the present invention is not particularly limited, and can be appropriately selected according to the purpose. For example, as the basic shape, a polygon (e.g., square, hexagon, octagon), circle, ellipse can be included. Preferably, it is a square or polygon.

The adhesive member of the present disclosure can be used as a base material for carrying a microneedle array to be described later. In that case, the microneedle array is carried on the surface of pressure-sensitive adhesive layer-side of the adhesive member of the present invention.

Further, the adhesive member of the present disclosure also includes an adhesive member, which has an opening portion at the center, wherein the opening portion has a size so as not to interfere with the passage of a microneedle array or a microneedle patch, and has a hollow raised portion (to be described later) protruding so as to cover the opening portion to a side where the adhesive layer is not set up, in the adhesive member of the present invention mentioned above. Hereinafter, such an adhesive member of the present invention will be referred to as the "adhesive member B of the present invention". In this case, the microneedle array is set up on the top surface of the hollow-inner surface of the raised portion of the adhesive member B of the present invention.

Here, "microneedle patch" means a patch in which a microneedle array is carried on an adhesive layer of an adhesive sheet.

2 Patch according to the present invention

A patch according to the present invention (hereinafter, referred to as the "patch of the present invention") is one in which a microneedle array is carried on a surface of adhesive layer-side of the adhesive member of the present invention (patch A of the present invention). Alternatively, the patch of the present invention is one in which a microneedle array, a microneedle patch, or the patch A of the present invention is set up on the top surface of hollow-inner surface of the raised portion of adhesive member B of the present invention (patch B of the present invention).

2.1 Microneedle and microneedle array

A microneedle array is one in which a substantial number of microneedles are mounted on a suitable flat plate. Such a microneedle is usually capable of carrying a medicament.

In the "microneedle array" of the patch of the present invention, microneedles with a height ranging from 100 to 1000 $\mu$m are set up on a suitable flat plate at 50 to 1000 per cm$^2$. If the number of microneedles is large, the amount of drug loaded can be increased. Preferred microneedle heights are within the range of 120 to 800 $\mu$m, more preferably within the range of 150 to 600 $\mu$m.

The material of the flat plate on which the microneedles are set up is not particularly limited, as long as microneedles can be stably set up. For example, the material can include metals, plastics, and ceramics. Also, it can include, for example, stainless steel, iron, titanium, polyesters, polycarbonates, polystyrenes, polyolefins, polyacrylics, polycycloolefins, silicon, polyethylenes, polypropylenes, polyvinyl chlorides.

The material of the microneedle array is not particularly limited, as long as it can puncture microneedles into the skin and carry drugs, and examples thereof include metal, plastic, and ceramic. Also, it can include, for example, stainless steel, iron, titanium, polyesters, polycarbonates, polystyrenes, polyolefins, polyacrylics, polycycloolefins, silicon. Among the polyesters, a polyglycolic acid-based biodegradable resin, a polylactic acid-based biodegradable resin, and the like are preferred from the viewpoint of safety because they gradually decompose in a living body.

The tip diameter of the microneedle is usually in the range of 1 to 20 $\mu$m. The tip diameter in the range of 1 to 10 $\mu$m is preferred to enhance the effectiveness of smoothly puncturing the epidermal layer of the patient's skin. On the other hand, when the tip diameter is more than 30 $\mu$m, the resistance at the time of puncturing the skin becomes large and is difficult to puncture the skin, and the tip is easily deformed, which is not preferable.

The shape of the microneedle can be appropriately selected according to the purpose, such as a cone, a pyramid, or a shape in which a cone is set up in the center of a truncated cone.

The planar shape of the microneedle array is not particularly limited, and can be appropriately selected according to the purpose. For example, as the basic shape, a polygon (e.g., square, hexagon, octagon), circle, ellipse can be included. Preferably, it is a square or polygon.

The pushing force is not particularly limited, as long as it is a force necessary in puncturing microneedles into the skin. If it is too large, pain is felt when pushing. So, it is preferably in the range of 1 to 200 N, more preferably in the range of 1 to 100 N. Since the pushing force is limited, a microneedle that punctures smoothly even under a small load is required. For example, it is preferable that the microneedles of 80% or more exhibit puncture property (e.g., a microneedle penetrates through the stratum corneum and is inserted into the skin) when the microneedle array is pushed 10 mm from the skin surface. In addition, when a force of 20 N or more is applied to a substrate having a diameter of 10 mm and pressed against the skin, it is preferable that the microneedles of 80% or more exhibit puncture property.

The microneedle or microneedle array according to the present disclosure can be manufactured according to a known manufacturing method. For example, it can be manufactured according to the manufacturing method described in WO2012/057345 or WO2013/162053.

2.2 Patch A of the present invention

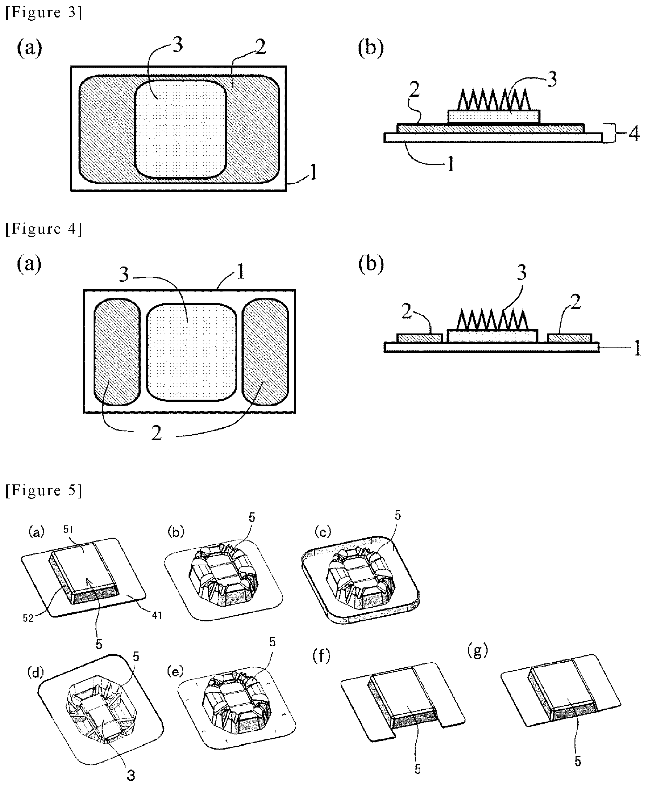

In the patch A of the present invention, a microneedle array is carried on a surface of adhesive layer-side of the adhesive member of the present invention. Specifically, for example, those of the embodiment shown in Figures 3 and 4 can be included.

In the adhesive member of the present disclosure having an adhesive layer on substantially the entire surface of one side of the support, the patch A of the present invention shown in Figure 3 carries a microneedle array at a center on the adhesive layer thereof. The patch A of the present invention shown in Figure 4 is an example of a patch having an adhesive layer on a part of the support. In the adhesive member of the present disclosure having a pair of adhesive layers on the left and right sides of one side of the support, a microneedle array is directly carried on the support between the pair of adhesive layers without via any adhesive layers.

The planar shape of the patch A of the present invention is not particularly limited and can be appropriately selected according to the purpose. For example, as the basic shape, a polygon (e.g., square, hexagon, octagon), circle, ellipse can be included. Preferably, it is a square or polygon.

According to the patch A of the present invention, when the patch is applied to the skin without stretching the skin, it is possible to suppress the floating of a microneedle array from the skin due to the elasticity of the skin. Further, according to the patch A of the present invention, when the skin is stretched and the patch is applied, it is possible to suppress floating of a microneedle array from the skin due to shrinkage of the skin or elasticity of the skin.

2.3 Patch B of the present invention

The patch B of the present invention is one in which a microneedle array, a microneedle patch, or the patch A is set up on the top surface of hollow-inner surface of the raised portion of adhesive member B of the present disclosure.

Specifically, for example, those of each embodiment shown in Figure 5 can be included. The opening portion may be a closed system surrounded by a periphery as shown in Figures 5(a) to (e), or as long as the effect of the present invention is not impaired, may be an open system in a partially open state as shown in Figures 5(f) and (g).

In one embodiment, the patch B of the present invention has the base portion (41) (corresponding to an adhesive member) containing a support and an adhesive layer, as shown in Figure 5 (a), and the hollow raised portion (5) which protrudes toward the side where the adhesive layer is not set up so as to cover an opening portion provided at the center of the base portion (41) thereof. The raised portion (5) includes the top surface (51) and the side wall (52). The side wall (52) is a surface connecting the base portion (41) and the ceiling. The adhesive layer is provided on at least a whole or a part of a surface of the base portion (41) to be applied to the skin. The adhesive layer may be further provided on the top surface (51) and/or the side wall (52). In the patch B of the present invention, both the base portion (41) and the raised portion (5) may be constituted with the adhesive member of the present invention.

The raised portion has an appearance as shown in Figure 5 and is a so-called "housing". The raised portion can be manufactured by heating and softening a film or a sheet extruded in advance, adhering the film or the sheet to a mold, and molding (vacuum molding) or molding by injecting a molten resin into a cavity in the mold (for example, injection molding). The raised portion may be formed of the same material as the base portion, and may be formed of different materials. A material suitable for molding the raised portion (housing) as described above can include a metal and a resin, and a material which is flexible and easily bends and deforms is more preferable, and for example, a metal such as aluminum or stainless steel; a resin such as polyethylene, polypropylene, polyethylene terephthalate, polystyrene, nylon, acrylic, silicon, or ABS can be included. When the microneedle is held on the skin without cutting off the microneedle array from the patch of the present invention after the microneedle is punctured, it is preferable that the support carrying the microneedle array is not restored, and a metal or a resin which is difficult to be restored is selected. In addition, a mechanism for preventing the restoration may be separately equipped. The thickness of each part of the housing is appropriately selected so that a desired deformation, which will be described later, occurs when a person presses the housing from above with a finger.

It is preferable that a crease or an edge for promoting deformation is formed on the top surface and/or the side wall. As shown in Figure 5, the "crease or edge" is formed so that when the top surface portion of the raised portion (5) is pressed by a finger or the like, the raised portion (5) is easily deformed, and the microneedle array set up inside the top surface can be pushed down to the skin. The "crease or edge" is usually set up radially from the top surface to the side wall. Further, it is preferable that the side wall is subjected to processing such as symmetrically installation so that the top surface can move vertically with respect to the skin.

Incidentally, in order to prevent deformation when uneven force is applied to the top surface or the base portion by a pressing operation with a finger or the like, uneven portions for reinforcement to increase the strength of the top surface and the base portion may be added.

The rigidity of patch B of the present invention is 1.3 MPa•mm$^4$ or more, preferably within the range of 1.3 MPa•mm$^4$ to 3400 MPa•mm$^4$, more preferably within the range of 1.3 MPa•mm$^4$ to 1200 MPa•mm$^4$, or within the range of

1. 3 MPa•mm$^4$ to 730 MPa•mm$^4$. The rigidity can be appropriately adjusted according to the thickness, the material, and the shape of the base portion; the materials and the shapes of the raised portion and the side wall; the number and the shapes of creases and edges provided on the top surface and/or the side wall; the shape of the opening portion, and the like.

A microneedle array may be internally set up on the top surface (51) of patch B of the present invention. In some embodiments, the microneedle array is set up directly on the adhesive layer constituting the top surface (51).

In some embodiments, a microneedle patch can be internally set up in the patch B of the present invention. Hereinafter, a microneedle patch set up internally in the patch B of the present invention may be generically referred to as the "internal patch". The internal patch may be the patch A of the present invention. Usually, the top surface and/or the side wall of hollow portion is deformed, so that the microneedle array can be moved toward the skin surface side.

The patch B of the present invention in which the patch A of the present invention is internally set up above the inner surface of raised portion is preferable. When the patch B of the present invention is applied to the skin without stretching the skin, the patch A of the present invention set up internally above the inner surface of raised portion makes it possible to suppress the floating of microneedle array from the skin due to the elasticity of the skin. In addition, when the skin is stretched and the patch B of the present invention is applied, and then the patch A of the present invention, which is internally set up above the inner surface of raised portion, is separated from the patch B of the present invention and is fixed to the skin, microneedles can be punctured into the skin while maintaining the extension of the skin, and the floating of microneedle array from the skin due to the contraction of the skin or the elasticity of the skin can be suppressed.

The internal patch may be fixed to the top surface (51) with an adhesive, or may be fixed by, for example, locking with a locking nail. The internal patch is usually easily detachably fixed to the patch B of the present invention.

When the internal patch is internally set up in the patch B of the present invention, after microneedles are punctured into the skin, usually, the internal patch is removed from the patch B of the present invention and is applied to the skin. It is preferable that the adhesive for fixing the internal patch to the patch B of the present invention is set up on the back side of the internal patch (a surface on which the microneedles do not protrude) and inside the end of the internal patch. In doing so, the transferability of the internal patch to the skin can be improved. This is because, after the skin puncture of microneedle, at the time when the internal patch is applied to the skin and is removed from the patch B of the present invention, a force to be peeled off is largely applied to the outermost portion of the adhesive portion in which the internal patch is fixed to the patch B of the present invention, which becomes a trigger point and allows the internal patch to easily peel off.

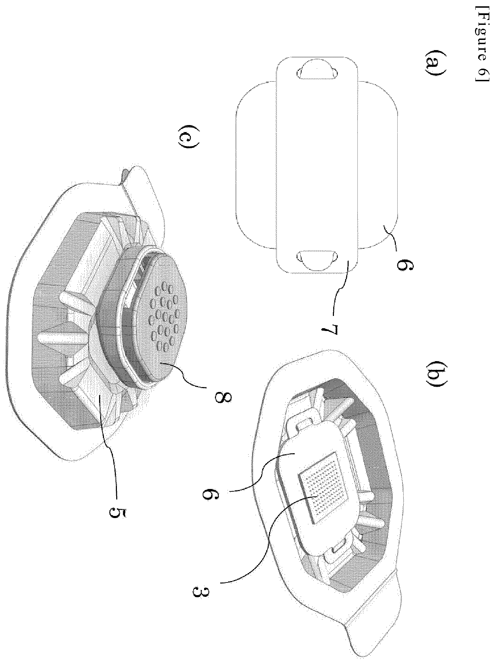

In the patch B of the present invention, an example of a case in which the internal patch is fixed with a locking nail is shown in Figure 6. In the figure, the figure (6) is the internal patch having a locking nail. Although a member for locking a locking nail is not particularly limited. In the example of Figure 6, the locking nail is locked with a locking hole, and the fitting member (7) having a locking hole is fixed to the top surface (51). The locking member such as the locking hole may be integrally and directly set up on the top surface (51) or the side wall (52).

When the skin is stretched and then the skin is fixed by applying the patch B of the present invention, the patch B of the present invention can be fixed without shrinking the skin. Also, the top and/or side walls are deformable (particularly (b) to (e) of Figure 5), and due to deformation, the top surface proceeds to the skin surface and microneedles can be inserted into the skin.

2.4 Others

The method of spreading the skin when using the patch of the present invention is not particularly limited, and for example, a method of spreading the skin by hand, a method of spreading the skin by using a suitable jig capable of spreading the skin, a method of spreading the skin with an elastic body such as rubber, a method of spreading the skin by bending the joint in a direction in which the skin is pulled, and the like can be given. Further, although there is no particular limitation on the method of moving and inserting the microneedle array into the skin by the patch of the present invention, for example, a method of pushing by hand, a method of inserting by injection or pressing using a spring (coiled, plate-like, dome-like, or the like), and the like can be included.

The patch of the present invention can be applied to humans, and also can be applied to other animals. The application site of the patch of the present invention is not particularly limited, and in the case of humans, for example, the medial and lateral sides of the forearm, the back of the hand, the medial and lateral sides of the upper arm, the shoulder, the back, the leg, the buttock, the abdomen, the chest, and the like can be included.

The meaning and the like of other terms related to the patch of the present invention are the same as those described above.

3 Auxiliary tool according to the present disclosure

An auxiliary tool according to the present disclosure (hereinafter referred to as the "auxiliary tool of the present disclosure") is an assisting device for puncturing microneedles into the skin, and is characterized in having an opening portion at the center in the adhesive member of the present disclosure, wherein the opening portion has a size so as not to interfere with the passage of a microneedle array or a microneedle patch.

The rigidity of auxiliary tool of the present disclosure is 1.3 MPa·mm$^4$ or more, but is preferably in the range of 1.3 MPa·mm$^4$ to 3400 MPa·mm$^4$, more preferably in the range of 1.3 MPa·mm$^4$ to 1200 MPa·mm$^4$, or in the range of 1.3 MPa·mm$^4$ to 730 MPa·mm$^4$. When the rigidity is lower than 1.3 MPa·mm$^4$, even if the skin is fixed with the auxiliary tool after stretching the skin, the skin may shrink, and it may not be successfully fixed even if an attempt is made to apply the microneedle patch. Of particular importance is that the rigidity has a rigidity of 1.3 MPa·mm$^4$ or more.

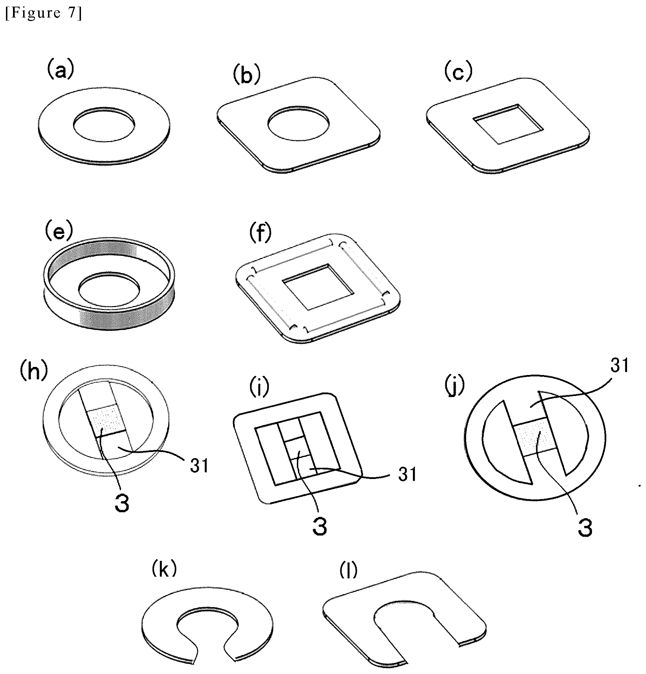

The auxiliary tool of the present disclosure is generally a ring-shaped tool having an opening portion at the center as shown in Figure 7(a), (b), (c) and the like. The shape of the opening portion is not particularly limited and is appropriately selected according to the strength and the puncture operation of the microneedle array. For example, circular, elliptical, polygonal (e.g., square, pentagonal, hexagonal, octagonal) shapes can be included. Among them, the shape of the circular or square is preferred.

In addition, the auxiliary tool of the present disclosure may have a form in which a part as shown in Figure 7(k) and (l), which is not ring-shaped, is missing as long as the effect of the present invention is not impaired.

Furthermore, in order to improve the rigidity, the auxiliary tool of the present disclosure may be provided with a peripheral wall which is suspended from the peripheral end as shown in Figure 7(e), or may be provided with a curved structure as shown in Figure 7(f).

The auxiliary tool of the present disclosure can prevent shrinkage of the skin when the microneedle patch is fixed to the skin surface in a state in which the skin is stretched. As a result, it is possible to prevent floating of the pasted microneedle array from the skin.

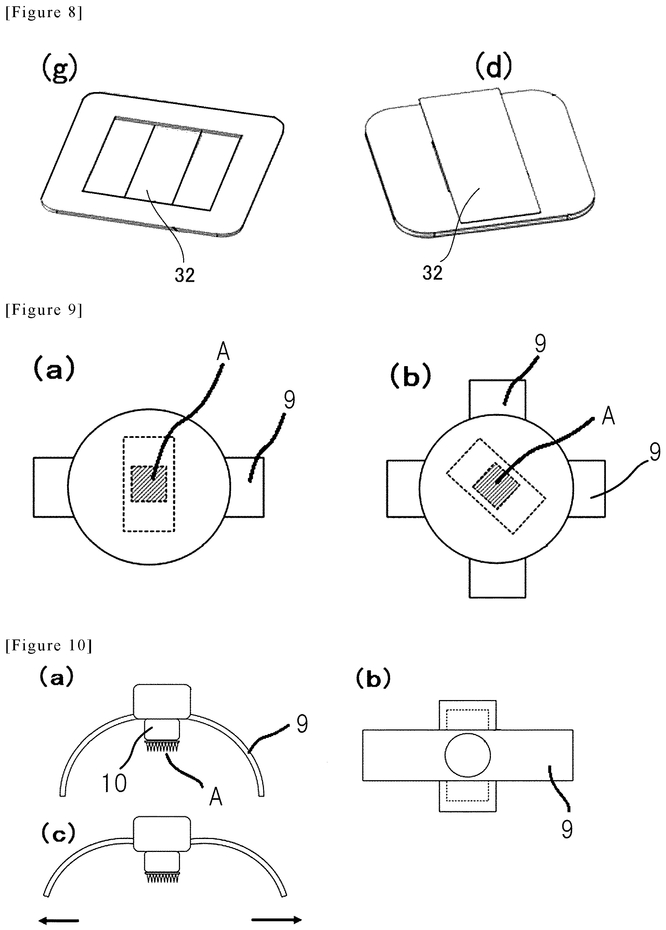

Figure 8 shows an example of the use of auxiliary tool of the present disclosure shown in Figure 7(c). In Figure 8(g), the opening portion has a size not to interfere with the passage of the microneedle patch (32). By crimping and sticking the microneedle array to the skin surface of the opening portion, microneedles can be inserted into the stretched skin. Specifically, after stretching the skin with a constant force, the skin is fixed in an extended state by sticking the auxiliary tool of the present invention to the skin on the skin surface while being stretched. Then, a microneedle array or a microneedle patch containing the microneedle array is inserted into the fixed stretched skin from the opening portion.

Figure 8(d) shows an opening portion having a size that prevents the microneedle patch (32) from passing therethrough, but does not interfere with the passage of a microneedle array. The microneedle patch (32) is previously fixed to an auxiliary tool of the present invention with an adhesive, a fixing jig, or the like, and the skin is stretched to maintain an extended state of the skin by applying the auxiliary tool of the present invention to the skin, and after enhancing the puncture property of the microneedle, a pressure is applied to the microneedle array, or the microneedle array is moved to the skin surface, so that the microneedle can be inserted into the skin.

In addition, a microneedle array or a microneedle patch may be set up in the opening portion of auxiliary tool of the present invention. For example, as shown in Figure 7(h), (i), and (j), the support member (31) of the microneedle array is set up in the opening portion, and the microneedle array (3) is set up on the support member (31). The support member (31) may be integrally formed with the auxiliary tool of the present disclosure by the same material as that of the auxiliary tool of the present disclosure. It is preferable that the support member (31) has sufficient flexibility so as to be able to move to the skin surface. The flexibility is adjusted by appropriately selecting the material, shape or thickness of the support member (31).

It is preferable that a microneedle patch to be applied to the skin using the auxiliary tool of the present disclosure is the patch A of the present invention. When the microneedle patch is applied to the skin without stretching the skin, if the microneedle patch is the patch A of the present invention, it is possible to suppress the floating of the microneedle array from the skin due to the elasticity of the skin. In addition, when the skin is stretched to fix the skin with the auxiliary tool of the present disclosure and the microneedle patch is applied to the skin, and then the microneedle patch is separated from the auxiliary tool of the present disclosure to fix to the skin, if the microneedle patch is the patch A of the present invention, the floating of microneedle array from the skin due to shrinkage of the skin or elasticity of the skin can be suppressed.

The method of spreading the skin when using the auxiliary tool of the present disclosure is not particularly limited, and for example, a method of spreading the skin by hand, a method of spreading the skin by using a suitable jig capable of spreading the skin, a method of spreading the skin with an elastic body such as rubber, and a method of spreading the skin by bending the joint in a direction in which the skin is pulled can be included. The method of moving and inserting the microneedle array into the skin using the auxiliary tool of the present disclosure is not particularly limited, and for example, a pushing method by hand, a method of injecting or pressing the microneedle array using a spring (a coil shape, a plate shape, a dome shape, or the like) to insert the microneedle array into the skin can be included.

The auxiliary tool of the present disclosure can be applied to humans, and also can be applied to other animals. The application site of the auxiliary tool of the present disclosure is not particularly limited, and in the case of humans, for example, the medial and lateral sides of the forearm, the back of the hand, the medial and lateral sides of the upper arm, the shoulder, the back, the leg, the buttock, the abdomen, the chest, and the like can be included.

The meaning and the like of other terms relating to the auxiliary tool of the present disclosure are the same as those described above.

4 Device according to the present disclosure

A device according to the present disclosure (hereinafter referred to as the "device of the present disclosure") is a device wherein the patch A of the present invention is set up in an applicator for transferring and applying a microneedle array to the skin or in an applicator for stretching the skin to transfer and apply a microneedle array to the skin. The device of the present disclosure usually equips some or all of the attachments necessary for drug administration, and can be referred to as a device for administering a medicament. Through an applicator according to the device of the present disclosure, the patch A (microneedle array) of the present invention can be effectively applied to the skin

The above-mentioned "applicator for transferring and applying a microneedle array to the skin", for example, can include one in the form shown in Figure 5 or 6. In addition, the "applicator for stretching the skin to transfer and apply a microneedle array to the skin" mentioned above, for example, can include those in the form shown in Figures 9 to 12. The applicator according to Figures 9 to 12 particularly has the skin stretching mechanism portion (9) for stretching the skin and the applying mechanism portion (10) for transferring and applying a microneedle array to the skin.

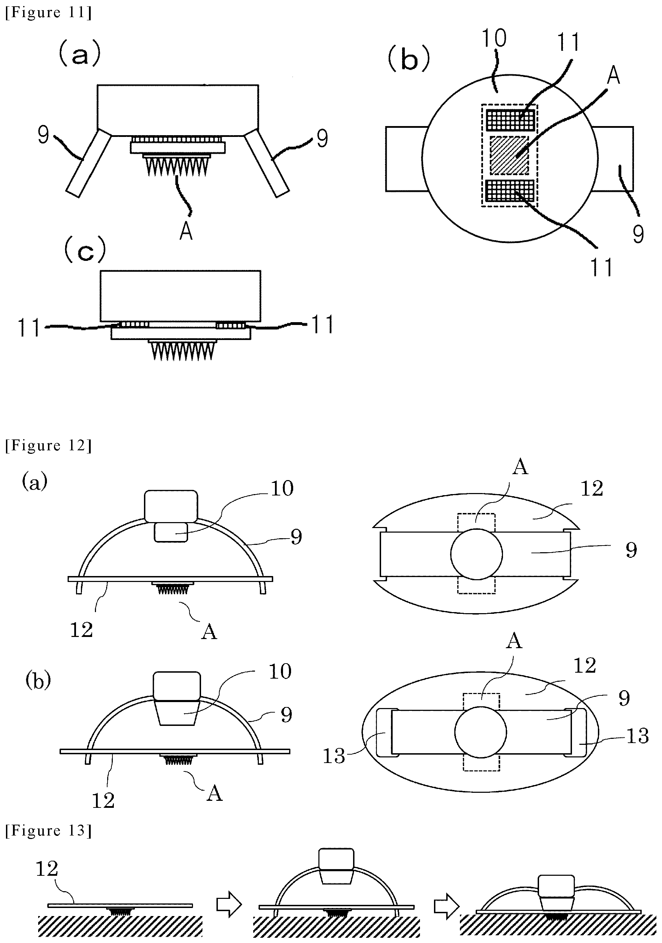

The patch A of the present invention is usually engaged or adhered to the applicator or the skin stretching mechanism portion (9) by means such as structually fitting together or the like, or with an adhesive or the like. Figure 11 illustrates a method for setting up the patch A of the present invention on the applicator with an adhesive. After puncturing microneedles into the skin, usually, the patch A of the present invention is removed from the applicator and applied to the skin, but it is preferable that the adhesive for fixing the patch A of the present invention to the applicator is installed on the back side of the patch A of the present invention (a surface on which the microneedles do not protrude) and inside the end of the patch A of the present invention. In doing so, it is possible to improve the transferability of the patch A of the present invention to the skin. This is because, after the skin puncture of the microneedle, at the time when the patch A of the present invention is applied to the skin and is removed from the applicator, a force to be peeled off is largely applied to the outermost portion of the adhesive portion (11) which fixes the patch A of the present invention to the applicator, which becomes a trigger point and allows the patch of the present invention to easily peel off.

In addition, as shown in Figure 12, the device of the present invention may have a structure in which the portion (12) having the patch A of the present invention is not directly adhered or engaged with the applicator or the skin stretching mechanism portion (9). In the device of the present disclosure having such a structure, for example, the portion (12) having the patch A of the present invention is set up without being crimped to the skin, and next the positioning may be adjusted by the shapes of the portion (12) having the patch A of the present invention and the skin stretching mechanism portion (9), and then the microneedle array is moved to and inserted into the skin stretched by the skin stretching mechanism portion (9) to be applied (see Figure 13). Examples of the shape of the portion (12) having the patch A of the present invention include a notched structure having both ends cut off (see right in Figure 12(a)), a structure having the cavity (13) at both end portions (see right in Figure 12(b)), and the like. Alternatively, the positioning may be simply adjusted visually.

The device of the present disclosure also includes one having a form in which an applicator having a hollow raised portion such as the patch B of the present invention and capable of moving and applying a microneedle array to the skin is combined with the patch A of the present invention. When the patch A of the present invention (serving as the internal patch) is fixed to the hollow portion, it may be fixed with an adhesive, or may be physically fixed by fitting with a protrusion or the like. After puncturing microneedles, usually, the patch A of the present invention is removed from the applicator to apply to the skin. Then, it is preferable that the adhesive for fixing the patch A of the present invention to the applicator of the present disclosure is installed on the back side of the patch A of the present invention (a surface on which the microneedle does not protrude) and inside the end of the patch A of the present invention. In doing so, it is possible to improve the transferability of the patch A of the present invention to the skin. This is because, after puncturing microneedles, at the time when the patch A of the present invention is applied to the skin and is removed from the applicator, a force to be peeled off is largely applied to the outermost portion of the adhesive portion which fixes the patch A of the present invention to the applicator, which becomes a trigger point and allows the patch of the present invention to easily peel off.

An example of the case of physically fixed by fitting with a protrusion or the like is shown in Figure 6. In the case of fixing by fitting, the fitting member (7) of the patch A (the internal patch (6)) of the present invention needs equal to or higher than a certain level in rigidity. If the rigidity is not appropriate, the fitting may be weak and may not be possible. The rigidity is preferably $1.3 \ MPa \cdot mm^4$ or more, more preferably in the range of $1.3 \ MPa \cdot mm^4$ to $3400 \ MPa \cdot mm^4$, more preferably in the range of $1.3 \ MPa \cdot mm^4$ to $1200 \ MPa \cdot mm^4$, or in the range of $1.3 \ MPa \cdot mm^4$ to $730 \ MPa \cdot mm^4$.

When the patch A (the internal patch (6)) of the present invention is fixed by fitting as in the example of Figure 6, the planar shape thereof is not particularly limited, but a protrusion, a dent, or a hollow need for fitting. It is preferable that each portion where the patch A (the internal patch (6)) of the present invention and the fitting member (7) are fitted together is not provided with an adhesive, and the patch A (the internal patch (6)) of the present invention and the fitting member (7) are not adhered. Further, the fitting member (7) may be glued or adhered to the raised portion (5) or may be integral therewith. It may also be the fitting member (7) by providing a protrusion, a dent, a hollow, or the like in the raised portion (5). Although Figure 6 shows an example of installation by fitting, the shape to fit is not particularly limited.

The device of the present disclosure may also be equipped with an indicator for sensing stress.

The device of the present disclosure can be applied to humans, and also can be applied to other animals. The application site of the device of the present disclosure is not particularly limited, and in the case of humans, for example, the medial and lateral sides of the forearm, the back of the hand, the medial and lateral sides of the upper arm, the shoulder, the back, the leg, the buttock, the abdomen, the chest, and the like can be included

The meaning and the like of other terms related to the device of the present invention are the same as those described above.

EXAMPLE

Hereinafter, the present disclosure will be illustrated in detail by examples and test examples, but the present invention is not limited to the ranges described in the examples and the like.

[Example 1] Correlation between rigidity and tape (patch) floating

(1) How to obtain rigidity

(1-1) Test method

In accordance with Japanese Industrial Standards (JIS K7171), by the three-point bending test shown in Figure 13, the rigidity of test pieces (equivalent to the support of adhesive of the present invention or the patch of the present invention. The same applies hereinafter.) was determined. Specific test methods are as follows.

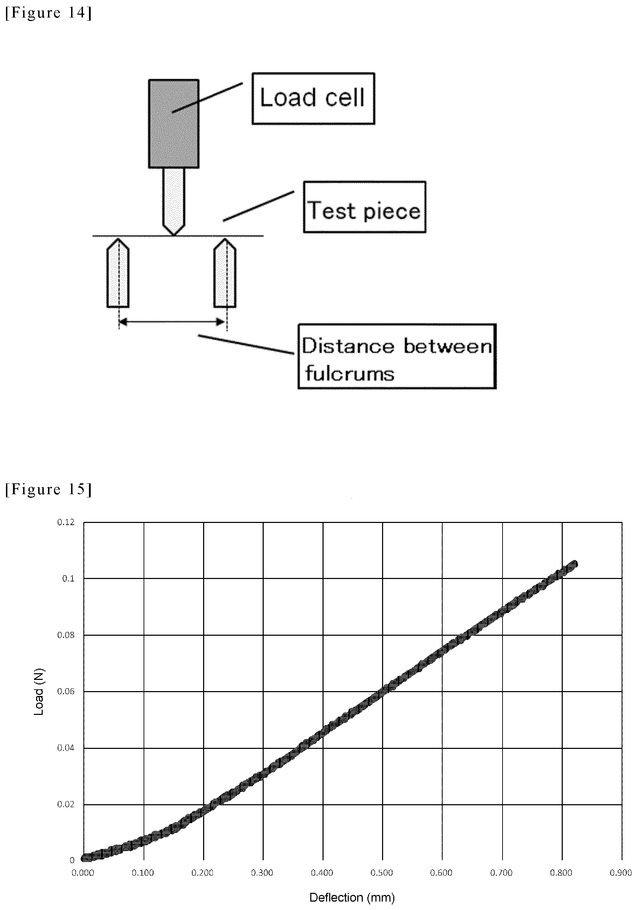

The test pieces were set up on two support stands, and the force was applied with an indenter to the central portion, deflected at a constant speed, and the force and deflection applied to the test pieces were measured. An example of the load curve of the obtained test result is shown in Figure 14.

Apparatus: EZTest of a small tabletop testing machine manufactured by Shimadzu Corporation
Test speed: 1 mm/min
Distance between fulcrums: 12 mm
Test piece: 10 to 20 mm width, 30 mm length

Of the test pieces, the one having a low rigidity was measured by attaching a 0.08 mm thick PET sheet (with a silicon coat) thereto, together with the PET sheet, as a test piece with a PET sheet. Then, it was also measured for the PET sheet alone, and the measured value was obtained by subtracting the numerical value of the PET sheet alone from the test piece with the PET sheet.

Test piece having low rigidity: Micropore (registered trademark, manufactured by 3M Company), Blenderm (registered trademark, manufactured by 3M Company), Transpore (registered trademark, manufactured by 3M Company), Durapore (registered trademark, manufactured by 3M Company), Porous Tape (registered trademark, manufactured by MILLION Co., Ltd.)

The PET made-tape was prepared by attaching a 0.1mm-thick double-sided tape (J0990 manufactured by Nitoms, Inc.) to PET sheets.

(1-2) Method of calculating the rigidity

The rigidity can be calculated by the following equation.

$$\text{Rigidity (K)} = \text{Flexural modulus (E)} \times \text{Second moment of area (I)}$$

The flexural modulus (E) and the Second moment of area (I) are obtained as follows.

(1-2-1) How to Calculate Flexural Modulus (E)

First, the deflection $s_1$ and $s_2$ corresponding to the flexural strain ($\varepsilon_{f1} = 0.0005$ and $\varepsilon_{f2} = 0.0025$) are calculated from the following Formula 1.

[Formula 1]

$$s_i = \frac{\varepsilon_{fi} \cdot L^2}{6h}$$

( i = 1 or 2)

$s_i$ : Deflection (mm)
$\varepsilon_{fi}$ : Flexural strain
L : Distance between fulcrums (mm)
h : Average thickness of test piece (mm)

The flexural stress ($\sigma_f$) is calculated using Formula 2 below. Incidentally, F is a value at the deflection $s_1$ and $s_2$ calculated by the above Formula 1, based on the result of the load curve.

[Formula 2]

$$\sigma_f = \frac{3FL}{2bh^2}$$

$\sigma_f$ : Flexural stress (MPa)
F : Force (N)
L : Distance between fulcrums (mm)
b : Average width of test piece (mm)
h : Average thickness of test piece (mm)

The flexural modulus ($E_f$, MPa) can be calculated by the following Formula 3, based on the calculated flexural stress ($\sigma_f$) and the like.

[Formula 3]

$$E_f = \frac{\sigma_{f2} - \sigma_{f1}}{\varepsilon_{f2} - \varepsilon_{f1}}$$

$\sigma_{f1}$ : Flexural stress measured at deflection $s_1$ (MPa)
$\sigma_{f2}$ : Flexural stress measured at deflection $s_2$ (MPa)

(1-2-2) How to obtain the second moment of area (I)

The second moment of area (I) can be calculated by the following Formula 4.

[Formula 4]

$$\text{Second moment of area } (I) = \frac{bh^3}{12}$$

(2) Evaluation of tape (patch) floating

(2-1) Evaluation method

A tape with a 10 mm x 10 mm x 1 mm-thick PET plate installed centrally instead of a microneedle array was pressed near the medial center of the human forearm at approximately 40 N to adhere to the forearm. After about 1 minute, the degree of tape float was visually observed. The criteria below were followed for evaluation.

Evaluation: × = Tape much floating, unsuitable

O = Tape seldom floating, good

◎ = No tape floating, very good

(2-2) Results

For each tape (patch), the correlation between the float and the rigidity is summarized. The results are shown in Table 1.

[Table 1]

| Tape | Tape width (mm) | Tape thickness (mm) | Tape floating | Rigidity (MPa · mm$^4$) |
|---|---|---|---|---|
| **Micropore** | 10 | 0.10 | × | 0.59 |
| **Blenderm** | 15 | 0.11 | × | 0.64 |
| **Porous Tape** | 15 | 0.18 | × | 0.88 |
| **Transpore** | 10 | 0.13 | × | 0.98 |
| **Durapore** | 10 | 0.18 | × | 1.2 |
| **PET** | 10 | 0.18 | ○ | 1.3 |
| **3 sheets of Micropore** | 10 | 0.30 | ○ | 1.6 |
| **Durapore** | 15 | 0.18 | ○ | 1.9 |
| **PET** | 15 | 0.18 | ◎ | 2.0 |
| **PET** | 20 | 0.18 | ◎ | 2.6 |
| **PET** | 15 | 0.3 | ◎ | 91 |
| **PET** | 15 | 0.4 | ◎ | 220 |
| **PET** | 15 | 0.5 | ◎ | 420 |
| **PET** | 15 | 0.6 | ◎ | 730 |
| **PET** | 21 | 0.6 | ◎ | 1000 |
| **PET** | 15 | 0.7 | ◎ | 1200 |
| **PET** | 15 | 1.0 | ◎ | 3400 |

As is apparent from the results of Table 1, in the tape (patch) having a rigidity of 1.2 MPa•mm$^4$ or less, the tape was poor in floating from the skin. In the case of the tape (patch) having higher rigidity, the floating of the tape was hardly observed, and it was good.

[Example 2] Relationship between rigidity and stress applied to a microneedle

(Evaluation of stress applied to a microneedle)

(1) Production of human skin model

A silicon sheet with a hardness of 5 degrees (15 mm thick) was used as a human skin model. Compared the elasticity of the model with that of the skin, stress curves drawn in both were equivalent.

(2) How to measure stress applied to a microneedle

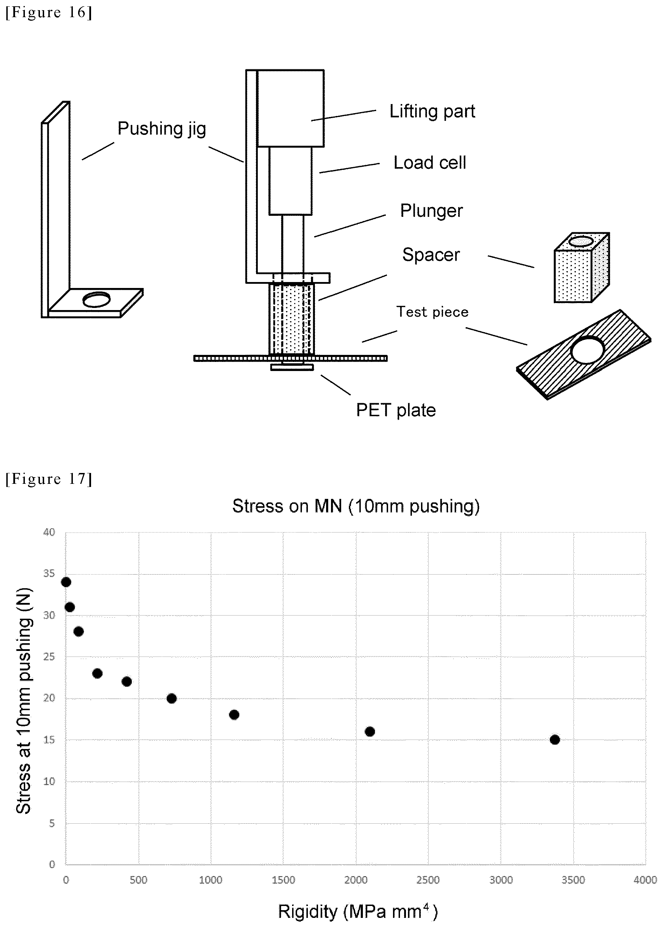

The measurement was performed using an apparatus as shown in Figure 16. In the lifting part of the apparatus, a load cell, a plunger of 5 mmφ, a spacer of 10 mm x 10 mm x 20 mm-height and a pushing jig is attached. The pushing jig is attached to the lifting part of the apparatus as shown in Figure 16, the spacer provided with a through hole of 8 mmφ, the plunger connected to the load cell passes through the hole.

On the human skin model, a PET plate of 10 mm x 10 mm x 1 mm-thick was set up as a pseudo microneedle array, and a test piece with a hole of 8 mm diameter was set up thereon. The plunger was then set up through a hole in the test piece to contact the top surface of the PET plate, which is an alternative to a microneedle array.

The lifting part was lowered by 10 mm, and the stress applied to the PET plate, which was an alternative to a microneedle array, was measured.

In the apparatus, the pushing jig attached to the lifting part presses down the test piece through the spacer, the PET plate is also depressed by the plunger in tune with the test piece is depressed. Since the load cell is connected to the PET plate via the plunger and is not joined to the test piece, the force that the test piece receives from the human skin model is not measured. Therefore, only the stress applied to the PET plate is measured in the measurement data. In this test, no adhesive layer was installed in the test piece and a simple PET plate was used as the test piece.

Apparatus: EZTest small tabletop tester manufactured by Shimadzu Corporation

Test speed: 60 mm/min

(3) Results

As shown in Figure 17, as the rigidity of the test piece increased, the stress applied to the PET plate (pseudo microneedle array) decreased. It was shown that the rigidity of the PET plate (pseudo microneedle array) needs to be 3400 MPa•mm$^4$ or less in order to apply a stress of 15 N or more to a microneedle array, and that of the PET plate (pseudo microneedle array) needs to be 730 MPa•mm$^4$ or less in order to apply a stress of 20 N or more to a microneedle array.

In the test piece with a hole of 8 mm in the center and the test piece without a hole, the stresses applied to the test piece, on the human skin model, were measured by pushing by 10 mm with a 10 mm x 10 mm x 20 mm spacer (no through hole), but no difference in stress was observed depending on the presence or absence of the hole in the test piece.

Industrial Applicability

The patch of the present invention, the device of the present disclosure, and the auxiliary tool of the present disclosure are useful for pharmaceuticals and the like because the puncturability of microneedle is improved, and a microneedle array can be firmly fixed to the skin, and drugs can be quantitatively administered without waste.

Explanation of symbols

A      Patch of the present invention
1      Support
2      Adhesive layer
3      Microneedle Array
4      Adhesive member
41      Base portion
5      Raised portion
51      Top surface
52      Side wall
6      Internal patch
7      Fitting member
8      Pressing part
9      Skin stretching mechanism portion
10      Applying mechanism portion
11      Adhesive portion
12      Portion having the patch A of the present invention
13      Cavity

**Claims**

**1.** A patch, comprising:

an adhesive member, which comprises a support made of a resin and a pressure-sensitive adhesive set up on one surface of the support, and

a microneedle array carried on a surface of the pressure-sensitive adhesive layer-side of the adhesive member, **characterised in that** the adhesive member has a rigidity of 1.3 MPa·mm$^4$ to 730 MPa·mm$^4$.

2. The patch according to claim 1, wherein the planar shape of at least one of the patch, the adhesive layer, and the microneedle array is polygonal, square, circular, or elliptical.

3. The patch according to any one of the preceding claims, wherein the resin making up the support is polyethylene terephthalate -PET-.

4. The patch, according to any one of the preceding claims, wherein:

the adhesive member further comprises an opening portion at the center, wherein the opening portion has a size so as not to interfere with the passage of the microneedle array and has a hollow raised portion protruding so as to cover the opening portion to a side where the adhesive layer is not set up, and

the microneedle array is set up on the top surface of the hollow-inner surface of the raised portion of the adhesive member.

5. The patch according to claim 4, wherein the top surface and/or the side wall of the raised portion is deformable, and the microneedle array is movable toward the skin surface side.

6. The patch according to claim 5, in which a crease or an edge for promoting deformation is formed on the top surface and/or the side wall.

7. A microneedle array device, comprising:

a patch according to any one of claims 1 to 6; and
an applicator, the applicator comprising:

an applying mechanism portion for transferring and applying the microneedle array to the skin; and
a pair of a skin stretching mechanism portions for stretching the skin across the applying mechanism portion.

8. The microneedle array device according to claim 7, wherein the patch is installed structurally engaged or adhesively bonded to the applying mechanism portion or the pair of skin stretching mechanism portions.

9. The microneedle array device according to claim 7, wherein the patch is not installed directly on either the applying mechanism portion or the pair of skin stretching mechanism portions.

10. The microneedle array device according to any one of claims 7 to 9, wherein the planar shape of at least one of the patch, the adhesive layer, and the microneedle array is polygonal, square, circular, or elliptical.

11. The microneedle array device according to any one of claims 7 to 10, wherein the resin making up the support is polyethylene terephthalate -PET-.

**Patentansprüche**

1. Pflaster umfassend:

- ein Klebeelement, das einen Träger, der aus einem Harz hergestellt ist, und einen druckempfindlichen Klebstoff, der auf einer Oberfläche des Trägers angebracht ist, umfasst,
- eine Mikronadelanordnung, die auf einer Oberfläche der druckempfindlichen Klebstoffseite des Klebeelements getragen wird, **dadurch gekennzeichnet, dass** das Klebelement eine Steifheit von 1,3 MPa·mm$^4$ bis 730 MPa·mm$^4$ aufweist.

2. Pflaster nach Anspruch 1,
wobei die ebene Form von mindestens einem der folgenden Elemente, dem Pflaster, der Klebstoffschicht und der Mikronadelanordnung polygonal, quadratisch, kreisförmig oder elliptisch ist.

3. Pflaster nach einem der vorherigen Ansprüche,
   wobei das den Träger ausbildende Harz Polyethylenterephthalat (PET) ist.

4. Pflaster nach einem der vorherigen Ansprüche,

   wobei das Klebeelement ferner einen Öffnungsabschnitt in der Mitte umfasst,
   wobei der Öffnungsabschnitt eine Größe aufweist, die den Durchgang der Mikronadelanordnung nicht behindert,
   und einen hohlen, erhöhten Abschnitt aufweist, der so hervorsteht, dass er den Öffnungsabschnitt an einer Seite
   abdeckt, an der die Klebstoffschicht nicht angebracht ist,
   wobei die Mikronadelanordnung auf der Oberseite der hohlen Innenfläche des erhöhten Abschnitts des
   Klebelements angebracht ist.

5. Pflaster nach Anspruch 4,
   wobei die Oberseite und/oder die Seitenwand des erhöhen Abschnitts verformbar ist und die Mikronadelanordnung in
   Richtung der Hautoberflächenseite bewegbar ist.

6. Pflaster nach Anspruch 5,
   wobei an der Oberseite und/oder der Seitenwand eine Falte oder eine Kante zur Förderung der Verformung geformt
   ist.

7. Mikronadelanordnungsvorrichtung, die ein Pflaster nach einem der Ansprüche 1-6 und einen Applikator umfasst,
   wobei der Applikator einen Aufbringmechanismusabschnitt zum Übertragen und Aufbringen der Mikronadelanordnung auf die Haut und ein Paar von Hautdehnungsmechanismusabschnitten zum Dehnen der Haut über den
   Aufbringmechanismusabschnitt umfasst.

8. Mikronadelanordnungsvorrichtung nach Anspruch 7,
   wobei das Pflaster strukturell verbunden mit oder klebend an dem Aufbringmechanismusabschnitt oder dem Paar
   von Hautdehnungsmechanismusabschnitten angebracht ist.

9. Mikronadelanordnungsvorrichtung nach Anspruch 7,
   wobei das Pflaster weder direkt auf dem Aufbringmechanismusabschnitt noch auf dem Paar von Hautdehnungsmechanismusabschnitten angebracht ist.

10. Mikronadelanordnungsvorrichtung nach einem der Ansprüche 7-9,
    wobei die planare Form von mindestens einem der folgenden Elemente, dem Pflaster, der Klebstoffschicht und der
    Mikronadelanordnung polygonal, quadratisch, kreisförmig oder elliptisch ist.

11. Mikronadelanordnungsvorrichtung nach einem der Ansprüche 7-10,
    wobei das den Träger ausbildende Harz Polyethylenterephthalat (PET) ist.


**Revendications**

1. Un patch, comprenant :

   un élément adhésif, qui comprend un support fait d'une résine et d'un adhésif sensible à la pression disposé sur
   une surface du support, et
   un réseau de micro-aiguilles porté sur une surface du côté couche d'adhésif sensible à la pression de l'élément
   adhésif, **caractérisé en ce que** l'élément adhésif a une rigidité de 1,3 MPa•mm$^4$ à 730 MPa•mm$^4$ .

2. Le patch selon la revendication 1, dans lequel la forme planaire d'au moins l'un du patch, de la couche adhésive, et du
   réseau de micro-aiguilles est polygonale, carrée, circulaire, ou elliptique.

3. Le patch selon l'une quelconque des revendications précédentes, dans lequel la résine composant le support est le
   polyéthylène téréphtalate -PET-.

**4.** Le patch, selon l'une quelconque des revendications précédentes, dans lequel :

l'élément adhésif comprend en outre une partie d'ouverture au centre, dans lequel la partie d'ouverture a une taille de manière à ne pas interférer avec le passage du réseau de micro-aiguilles et a une partie surélevée creuse faisant saillie de manière à couvrir la partie d'ouverture vers un côté où la couche adhésive n'est pas disposée, et le réseau de micro-aiguilles est disposé sur la surface supérieure de la surface intérieure creuse de la partie surélevée de l'élément adhésif.

**5.** Le patch selon la revendication 4, dans lequel la surface supérieure et/ou la paroi latérale de la partie surélevée est déformable, et le réseau de micro-aiguilles est mobile vers le côté surface de peau.

**6.** Le patch selon la revendication 5, dans lequel un pli ou un bord pour favoriser la déformation est formé sur la surface supérieure et/ou la paroi latérale.

**7.** Un dispositif à réseau de micro-aiguilles, comprenant :

un patch selon l'une quelconque des revendications 1 à 6 ; et
un applicateur, l'applicateur comprenant :

une partie mécanisme d'application pour transférer et appliquer le réseau de micro-aiguilles sur la peau ; et
une paire de parties mécanisme d'étirement de la peau pour étirer la peau à travers la partie mécanisme d'application.

**8.** Le dispositif à réseau de micro-aiguilles selon la revendication 7, dans lequel le patch est installé structurellement en prise ou lié de manière adhésive à la partie mécanisme d'application ou à la paire de parties mécanisme d'étirement de la peau.

**9.** Le dispositif à réseau de micro-aiguilles selon la revendication 7, dans lequel le patch n'est pas installé directement sur ni la partie mécanisme d'application ni la paire de parties mécanisme d'étirement de la peau.

**10.** Le dispositif à réseau de micro-aiguilles selon l'une quelconque des revendications 7 à 9, dans lequel la forme planaire d'au moins l'un du patch, de la couche adhésive, et du réseau de micro-aiguilles est polygonale, carrée, circulaire, ou elliptique.

**11.** Le dispositif à réseau de micro-aiguilles selon l'une quelconque des revendications 7 à 10, dans lequel la résine composant le support est le polyéthylène téréphtalate -PET-.

[Figure 1]

(A)　　　　　　　　　　　　　　　　(B)

[Figure 2]

(A)　　　　　　　　　　　　　　　　(B)

[Figure 3]

(a)

(b)

[Figure 4]

(a)

(b)

[Figure 5]

(a) (b) (c)

(d) (e) (f) (g)

[Figure 6]

(a)

(b)

(c)

[Figure 7]

(a)

(b)

(c)

(e)

(f)

(h)
31
3

(i)
31
3

(j)
31
3

(k)

(l)

[Figure 8]

(g)

(d)

32

32

[Figure 9]

(a)

A

9

(b)

9

A

9

[Figure 10]

(a)

9

10

A

(c)

(b)

9

[Figure 11]

(a)

(b)

(c)

[Figure 12]

(a)

(b)

[Figure 13]

[Figure 14]

[Figure 15]

[Figure 16]

[Figure 17]

Stress on MN (10mm pushing)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009273872 A
- JP WO2012128363 A
- JP 2010516337 A
- JP 2014042788 A
- EP 3251721 A1
- WO 2012057345 A
- WO 2013162053 A